Europäisches Patentamt

European Patent Office

Office européen des brevets

(19)

(11) Veröffentlichungsnummer: **0 316 729 B1**

(12) **EUROPÄISCHE PATENTSCHRIFT**

(45) Veröffentlichungstag der Patentschrift: **29.07.92**

(51) Int. Cl.⁵: **C07D 311/72**

(21) Anmeldenummer: **88118624.1**

(22) Anmeldetag: **09.11.88**

(54) **Verfahren zum Anreichern von Tocopherolen in natürlichen Ouellen.**

(30) Priorität: **19.11.87 CH 4509/87**

(43) Veröffentlichungstag der Anmeldung:
**24.05.89 Patentblatt 89/21**

(45) Bekanntmachung des Hinweises auf die
Patenterteilung:
**29.07.92 Patentblatt 92/31**

(84) Benannte Vertragsstaaten:
**AT BE CH DE FR GB IT LI NL**

(56) Entgegenhaltungen:

**keine Entgegenhaltungen**

(73) Patentinhaber: **F. HOFFMANN-LA ROCHE AG**
**Postfach 3255**
**CH-4002 Basel(CH)**

(72) Erfinder: **Fizet, Christian, Dr.**
**18 rue de Bruebach**
**F-68440 Zimmersheim(FR)**

(74) Vertreter: **Cottong, Norbert A. et al**
**Grenzacherstrasse 124 Postfach 3255**
**CH-4002 Basel(CH)**

**Beschreibung**

Die vorliegende Erfindung betrifft ein neues Verfahren zum Aufkonzentrieren von natürlichen Tocopherolen und/oder von Steroiden in pflanzlichen Quellen.

Der Gewinnung von natürlichen Tocopherolen aus pflanzlichen Quellen kommt zunehmende Bedeutung zu, und es sind auch bereits vielerlei Verfahren zu diesem Zweck bekannt. Auch der Gewinnung von gewissen Steroiden aus derartigen Quellen kommt zunehmende Bedeutung zu.

Der Ausdruck natürliche Tocopherole umfasst, im Rahmen der vorliegenden Erfindung, nicht nur die α-, β-, γ- und δ-Tocopherole, sondern auch die entsprechenden Tocotrienole, Tocodienole und Tocoenole.

Als pflanzliche Ausgangsmaterialien für die Gewinnung von natürlichen Tocopherolen bzw. von gewissen Steroiden kommen, im Rahmen der vorliegenden Erfindung, insbesondere in Frage die im Zusammenhang mit der Gewinnung von Speiseölen- und Fetten nach der Desodorierungsstufe erhaltenen Destillate (Brüdenabscheiderfette). Insbesondere kommen hier in Frage die Rückstände der Desodorierung der üblichen Speiseöle und Fette, wie z.B. Sonnenblumenöl-Destillate, Sojaöl-Destillate, Rapsöl-Destillate, Baumwollsaatöl-Destillate, Palmöl-Destillate usw. Es können jedoch auch die noch nicht desodorierten Oele und Fette selbst verwendet werden, sowie auch die erwähnten Rückstände, welche bereits einer weiteren Konzentrationsstufe unterworfen wurden.

Diese Ausgangsprodukte enthalten in der Regel die Tocopherole sowie freie Fettsäuren und Glyceride von allen Säuren die normalerweise in Oelen und Fetten vorkommen können, Salze von Fettsäuren, Steroide und einen Rest von z.T. nicht identifizierbaren Begleitstoffen. Der mengenmässige Anteil dieser Substanzen variiert in der Regel sehr stark je nach Art der Ausgangsmaterialien und deren Gewinnungsverfahren. In den vorhergehend erwähnten Rückständen der Desodorierung liegt der Anteil an Tocopherolen in der Regel zwischen etwa 2 und etwa 15 Gew.-% und derjenige von Steroiden zwischen etwa 4 und etwa 15 Gew.-%. Der Gesamtanteil an freien Fettsäuren, Glyceriden und Salzen beträgt in der Regel von etwa 50 bis etwa 85 Gew.-% und derjenige des z.T. nicht identifizierbaren Restes von etwa 5 bis etwa 20 Gew.-%. In noch nicht desodorierten Fetten und Oelen, bzw. in bereits weiter aufkonzentrierten Rückständen der Desodorierung, liegen diese Werte selbstverständlich entsprechend niedriger bzw. höher. Diese Mengen sind vom Fachmann jeweils leicht zu ermitteln.

Im Hinblick auf die verhältnismässig geringe Menge an Tocopherolen bzw. Steroiden in den Ausgangsmaterialien im Vergleich zur Menge der restlichen Verbindungen, ist es wünschenswert, die Tocopherole und/oder die Steroide zunächst in den Ausgangsmaterialien anzureichern.

Diese Anreicherung kann z.B. dadurch erfolgen, dass man die Begleitstoffe, d.h. insbesondere die freien Fettsäuren und die Glyceride abtrennt. Dies erfolgt erfindungsgemäss nun dadurch, dass man die die anzureichernden Substanzen enthaltenden Ausgangsmaterialien mit Calciumhydroxid in Gegenwart von Wasser in einem mit Wasser mischbaren, inerten organischen Lösungsmittel behandelt und die so gebildeten Calciumsalze abtrennt.

Als mit Wasser mischbare organische Lösungsmittel, kommen im Rahmen der vorliegenden Erfindung insbesondere in Frage, niedere Alkohole mit 1 bis 6 Kohlenstoffatomen wie z.B. Methanol, Aethanol, Propanol, Isopropanol und dergleichen.

Durch die erwähnte Zugabe von Calciumhydroxid werden die freien Fettsäuren in Calciumsalze übergeführt. Dadurch, dass diese Reaktion in Gegenwart von Wasser durchgeführt wird, werden zudem die vorhandenen Glyceride unter Bildung von Fettsäure-Calciumsalzen verseift. Die Abtrennung der so erhaltenen Calciumsalze erfolgt zweckmässig durch Entfernen des verwendeten, mit Wasser mischbaren organischen Lösungsmittels und Ausfällung aus einem geeigneten weiteren Lösungsmittel. Als geeignete weitere Lösungsmittel kommen, im Rahmen der vorliegenden Erfindung, solche in Frage, welche normalerweise zur Auflösung von Fettsäure-Calciumsalzen nicht geeignet sind. Als Beispiele hierfür können insbesondere genannt werden Methyl- oder Aethylformiat- oder acetat, sowie auch Ketone oder Nitrile, und zwar insbesondere leichtflüchtige Vertreter hiervon, wie etwa Aceton, Acetonitril und dergleichen.

Die verwendete Menge an Calciumhydroxid in dem erfindungsgemässen Verfahren ist nicht kritisch und richtet sich nach der Menge an vorhandenen Fettsäuren und Glyceriden und kann vom Fachmann ohne weiteres festgelegt werden.

Die verwendete Menge an Wasser in dem erfindungsgemässen Verfahren liegt zweckmässig zwischen etwa 3 und etwa 15 Gew.-%, vorzugsweise bei etwa 6 bis 8 Gew.-% bezogen auf das verwendete Lösungsmittel.

Die Behandlung der Ausgangsmaterialien mit dem Calciumhydroxid erfolgt zweckmässig bei einer Temperatur von etwa 60°C bis etwa 110°C, vorzugsweise bei Rückflusstemperatur des Reaktionsgemisches. Je nach verwendetem Lösungsmittel muss u.U. in einem Druckbehälter (Autoklav) gearbeitet werden, um die erwähnten Temperaturen zu ermöglichen.

Durch das erfindungsgemässe Verfahren gelingt es in einfacher Weise, in einer einzigen Stufe, sowohl die im Ausgangsmaterial enthaltenen Fettsäuren als auch die Glyceride abzutrennen (durch einfaches Abfiltrieren oder Abzentrifugieren der entsprechenden Calciumsalze) und somit die vorhandenen Tocopherole und/oder Steroide um einen Faktor von etwa 2 bis etwa 6, je nach Art des verwendeten Ausgangsmaterials aufzukonzentrieren.

Aus den so erhaltenen Konzentraten können anschliessend die Tocopherole und/oder die Steroide in an sich bekannter Weise, z.B. durch Extraktion, Destillation, Kristallisation usw. gewonnen werden.

Beispiel 1

Als Ausgangsmaterial wird ein Sonnenblumenöl-Destillat folgender Zusammensetzung verwendet:

|  | Gew.-% |
|---|---|
| Tocopherole | 2,5 |
| Fettsäuren-Calcium-Salze | 25 |
| Fettsäuren | 36 |
| Glyceride | 15 |
| Wasser | 3 |
| Rest | 18,5 |

In einem Vierhals-Sulfierkolben, versehen mit Rückflusskühler, Thermometer und Rührer, werden unter Argonbegasung 100 g des obigen Destillates in 750 ml Isopropanol aufgenommen. Nach Zugabe von 50 ml Wasser und 10 g Calciumhydroxid wird das Gemisch 6 Stunden bei Rückflusstemperatur gerührt. Das Lösungsmittel wird anschliessend am Rotationsverdampfer entfernt und der Rückstand in 750 ml Essigsäureäthylester aufgenommen. Die erhaltene Suspension wird 1/2 Stunde bei Raumtemperatur gerührt und dann auf 0°C abgekühlt. Der Feststoff wird sodann abgenutscht und mit 100 ml Essigsäure-äthylester nachgewaschen. Das Filtrat wird am Rotationsverdampfer vollständig eingeengt und man erhält 22 g eines Rückstandes mit 10,8% Tocopherolen (95% Ausbeute).

Beispiel 2

Als Ausgangsmaterial wird ein Sojaöl-Destillat folgender Zusammensetzung verwendet:

|  | Gew.-% |
|---|---|
| Tocopherole | 9 |
| Fettsäuren-Calcium-Salze | 2 |
| Fettsäuren | 39 |
| Glyceride | 32 |
| Wasser | 1 |
| Rest | 17 |

In einem Vierhals-Sulfierkolben, versehen mit Rückflusskühler, Thermometer und Rührer, werden unter Argonbegasung 100 g des obigen Destillates in zu Beispiel 1 analoger Weise verarbeitet. Man erhält 26,3 g eines Rückstandes mit 31% Tocopherolen (90% Ausbeute).

Beispiel 3

Als Ausgangsmaterial wird ein Sonnenblumenöl-Destillat folgender Zusammensetzung verwendet:

|  | Gew.-% |
|---|---|
| Tocopherole | 2,4 |
| Fettsäuren-Calcium-Salze | 20 |
| Fettsäuren | 43 |
| Glyceride | 13 |
| Wasser | 4 |
| Rest | 17,6 |

In einem Vierhals-Sulfierkolben, versehen mit Rückflusskühler, Thermometer und Rührer, werden unter Argonbegasung 100 g des obigen Destillates in zu Beispiel 1 analoger Weise verarbeitet, mit dem Unterschied, dass der Rückstand nicht in Essigsäure-äthylester sondern in Essigsäure-Methylester aufgenommen wird. Man erhält 21,5 g eines Rückstandes mit 10,6% Tocopherolen (95% Ausbeute).

Beispiel 4

Als Ausgangsmaterial wird ein Palmöl-Destillat folgender Zusammensetzung verwendet:

|  | Gew.-% |
|---|---|
| Tocopherole (Tocopherole + Tocotrienole) | 1,1 % |
| Fettsäuren | 64 % |
| Glyzeride | 15 % |
| Rest | 19,9 % |

In einem Vierhals-Sulfierkolben, versehen mit Rückflusskühler, Thermometer und Rührer, werden unter Argonbegasung 100 g des obigen Materials in 600 ml Isopropanol aufgenommen. Nach Zugabe von 50 ml Wasser und 12 g Calciumhydroxyd wird das Gemisch 4 Stunden bei Rückflusstemperatur gerührt. Das Lösungsmittel wird anschliessend am Rotationsverdampfer entfernt und der Rückstand in 600 ml Essigsäuremethylester aufgenommen. Die erhaltene Suspension wird 1 Stunde bei Raumtemperatur gerührt. Der Feststoff wird sodann abgenutscht und mit 300 ml Essigsäure-methylester nachgewaschen. Das Filtrat wird am Rotationsverdampfer vollständig eingeengt und man erhält 18,1 g eines Rückstandes mit 5,2% Tocopherolen (Tocopherole + Tocotrienole) (86% Ausbeute).

Beispiel 5

Als Ausgangsmaterial wird ein Tocopherol-Konzentrat folgender Zusammensetzung verwendet:

|  | Gew.-% |
|---|---|
| Tocopherole | 19,5 |
| Fettsäuren | 38 |
| Fettsäuren-Ester | 2 |
| Glyceride | 2 |
| Rest | 38,5 |

In einem Vierhals-Sulfierkolben, versehen mit Rückflusskühler, Thermometer und Rührer, werden unter Argonbegasung 100 g des obigen Konzentrates in 750 ml Isopropanol aufgenommen. Nach Zugabe von 50 ml Wasser und 12,5 g Calciumhydroxyd wird das Gemisch 4 Stunden bei Rückflusstemperatur gerührt. Das Lösungsmittel und das Wasser werden anschliessend am Rotationsverdampfer entfernt und der Rückstand in 750 ml Essigsäuremethylester aufgenommen. Die erhaltene Suspension wird 1 Stunde bei Raumtemperatur gerührt. Der Feststoff wird sodann abgenutscht und mit 100 ml Essigsäuremethylester nachgewaschen. Das Filtrat wird am Rotationsverdampfer vollständig eingeengt und man erhält 49,5 g Rückstand mit 37,9% Tocopherolen (96% Ausbeute).

**Patentansprüche**

4

1. Verfahren zum Aufkonzentrieren von natürlichen Tocopherolen und/oder von Steroiden in diese Substanzen enthaltenden Ausgangsmaterialien, dadurch gekennzeichnet, dass man letztere mit Calciumhydroxid in Gegenwart von Wasser in einem mit Wasser mischbaren, inerten organischen Lösungsmittel behandelt und die so gebildeten Calciumsalze abtrennt.

2. Verfahren gemäss Anspruch 1, dadurch gekennzeichnet, dass man als Lösungsmittel einen niederen Alkohol mit 1 bis 6 Kohlenstoffatomen, vorzugsweise Methanol, Aethanol, Propanol oder Isopropanol verwendet.

3. Verfahren gemäss Anspruch 1 oder 2, dadurch gekennzeichnet, dass man die Reaktion in Gegenwart von etwa 3 bis etwa 15, vorzugsweise von etwa 6 bis etwa 8 Gew.-% Wasser, bezogen auf das verwendete Lösungsmittel, durchführt.

4. Verfahren gemäss einem der Ansprüche 1 bis 3, dadurch gekennzeichnet, dass man die Reaktion bei einer Temperatur von etwa 60°C bis etwa 110°C, vorzugsweise bei Rückflusstemperatur des Reaktionsgemisches, gegebenenfalls unter Druck, durchführt.

5. Verfahren gemäss einem der Ansprüche 1 bis 4, dadurch gekennzeichnet, dass man ein Ausgangsmaterial auf Basis von Sonnenblumenöl, Sojaöl, Rapsöl, Baumwollsaatöl oder Palmöl verwemdet.

## Claims

1. A process for the concentration of natural tocopherols and/or of steroids in starting materials containing these substances, characterized by treating the latter with calcium hydroxide in the presence of water in a water-miscible inert organic solvent and separating the thus-formed calcium salts.

2. A process in accordance with claim 1, characterized in that a lower alcohol with 1 to 6 carbon atoms, preferably methanol, ethanol, propanol or isopropanol, is used as the solvent.

3. A process in accordance with claim 1 or 2, characterized in that the reaction is carried out in the presence of about 3 to about 15, preferably from about 6 to about 8, wt.% of water based on the solvent used.

4. A process in accordance with any one of claims 1 to 3, characterized in that the reaction is carried out at a temperature of from about 60°C to about 110°C, preferably at the reflux temperature of the reaction mixture, optionally under pressure.

5. A process in accordance with any one of claims 1 to 4, characterized in that a starting material based on sunflower oil, soja oil, rape oil, cotton seed oil or palm oil is used.

## Revendications

1. Procédé pour concentrer des tocophérols naturels et/ou des stéroïdes dans des matières premières contenant ces substances, caractérisé en ce que l'on traite ces matières avec de l'hydroxyde de calcium en présence d'eau dans un solvant organique inerte miscible à l'eau, et en ce que l'on sépare les sels de calcium ainsi formés.

2. Procédé selon la revendication 1, caractérisé en ce que l'on utilise comme solvant un alcool inférieur ayant de 1 à 6 atomes de carbone, de préférence le méthanol, l'éthanol, le propanol ou l'isopropanol.

3. Procédé selon la revendication 1 ou la revendication 2, caractérisé en ce que l'on met en oeuvre la réaction en présence d'environ 3 à environ 15, de préférence d'environ 6 à environ 8% en poids d'eau, par rapport au solvant utilisé.

4. Procédé selon l'une des revendications 1 à 3, caractérisé en ce que l'on met en oeuvre la réaction à une température d'environ 60°C à environ 110°C, de préférence à la température de reflux du mélange réactionnel, éventuellement sous pression.

**5.** Procédé selon l'une des revendications 1 à 4, caractérisé en ce que l'on utilise une matière première à base d'huile de tournesol, d'huile de soja, d'huile de colza, d'huile de coton ou d'huile de palme.